# EUROPÄISCHE PATENTSCHRIFT

(11) **EP 3 972 490 B1**
(45) Veröffentlichungstag und Bekanntmachung des Hinweises auf die Patenterteilung: **23.07.2025**
(21) Anmeldenummer: 20740516.8
(22) Anmeldetag: 22.05.2020
(51) Int. Cl.: A61B 5/01, A61B 5/00, A43B 3/34, A43B 13/00, A43B 13/02, A43B 17/00

(54) **SENSORSCHICHT ZUM ERMITTELN VON TEMPERATURPROFILEN AN EINE HAUTOBERFLÄCHE, HILFSMITTEL ZUM ANLEGEN AN EINE HAUTOBERFLÄCHE,VERFAHREN ZUM HERSTELLEN EINES HILFSMITTELS UND VERFAHREN ZUM ERMITTELN EINER RELATIVEN TEMPERATURDIFFERENZ AN EINER HAUTOBERFLÄCHE**
SENSOR LAYER FOR DETERMINING TEMPERATURE PROFILES ON A SKIN SURFACE, AID FOR APPLICATION TO A SKIN SURFACE, METHOD FOR PRODUCING AN AID, AND METHOD FOR DETERMINING A RELATIVE TEMPERATURE DIFFERENCE ON A SKIN SURFACE
COUCHE DE CAPTEUR POUR DÉTERMINER DES PROFILS DE TEMPÉRATURE SUR UNE SURFACE DE PEAU, MOYEN AUXILIAIRE À APPLIQUER SUR UNE SURFACE DE PEAU, PROCÉDÉ POUR PRODUIRE UN MOYEN AUXILIAIRE ET PROCÉDÉ POUR DÉTERMINER UNE DIFFÉRENCE DE TEMPÉRATURE RELATIVE AU NIVEAU D'UNE SURFACE DE PEAU

(30) Priorität: 23.05.2019 DE 102019113719; 14.08.2019 DE 102019121927
(43) Veröffentlichungstag der Anmeldung: 30.03.2022
(73) Patentinhaber: Orthopädie-Technik-Service aktiv GmbH, 17489 Greifswald (DE)
(72) Erfinder: ESCHENBURG, Christian, 17489 Greifswald (DE); SCHEIBELER, Alf-Matthes, 17094 Holldorf (DE); TAUSCH, Günther, 17099 Datzetal (DE); STARKOWSKI, Frank, 17509 Lubmin (DE)
(74) Vertreter: Weidner Stern Jeschke
(86) Internationale Anmeldenummer: PCT/DE2020/200037
(87) Internationale Veröffentlichungsnummer: WO 2020/233756

(56) Entgegenhaltungen:
- WO-A2-2019/063488
- CN-A- 103 385 699
- US-A1- 2018 256 100
- US-B1- 10 080 524

## Beschreibung

Die Erfindung betrifft eine Sensorschicht zum Ermitteln von Temperaturprofilen an einer Hautoberfläche mit einer Kontaktschicht zur Hautoberfläche, wobei die Sensorschicht mindestens eine Lage 105) aufweist und auf einer Oberseite der mindestens einen Lage die Kontaktschicht angeordnet ist. Des Weiteren betrifft die Erfindung ein Hilfsmittel zum Anlegen an eine Hautoberfläche, ein Verfahren zum Herstellen eines Hilfsmittels und ein Verfahren zum Ermitteln einer relativen Temperaturdifferenz an einer Hautoberfläche.

Ein tiefliegender Substanzdefekt (Ulkus) und oberflächlicher Substanzdefekt (Wunde) der Haut werden üblicherweise erst wahrgenommen, wenn aufgrund einer Entzündung eine Rötung der Haut auftritt. Häufig wird eine Entzündung, insbesondere bei vorbelasteten Patienten, wie Diabetikern oder bei Bettlägerigkeit, zu spät wahrgenommen, welches eine schwer behandelbare Wundbildung, Gewebeschäden und/oder sogar eine Amputation zur Folge hat.

Bei einer für Substanzdefekte anfälligen Haut, wie dies bei Diabetikern oder bettlägerigen Personen gegeben ist, besteht zudem das Problem, dass die Haut von vornherein anfällig gegenüber Reizung und/oder Druck ist. Aus diesem Grunde können starre Messsensoren und/oder die zugehörige Messsensorik nicht direkt in Kontakt zur ohnehin schon anfälligen Hautoberfläche gebracht werden. Zudem ist häufig eine Überwachung von Körperstellen notwendig, welche bereits aufgrund des Körpergewichtes einer Druckbelastung ausgesetzt sind, wie beispielsweise die Fußsohlen oder der Rücken bei einem bettlägerigen Patienten.

Aus der CN 103385699 A ist eine resistive Temperatursensoranordnung bekannt, bei welcher temperatursensitive Filme auf einem flexibler Substratfilm angeordnet sind und der flexible Substratfilm auf einem festen Substrat, wie einer Leiterplatte oder Glasplatte, fixiert ist. Zwar kann der flexible Substratfilm auf eine Hautoberfläche aufgebracht werden, bei Belastung oder Bewegung kommt es jedoch aufgrund des festen Substrates zu Druckstellen an der Hautoberfläche.

Ebenso sind eine Druckausübung auf eine Hautoberfläche bei einer dreidimensionalen elektronischen Kompresse der US 2017/0007133 A1 sowie einer tragbaren Kompresse der US 2018/0184908 A1 mit zwei Temperatursensor angeordnet in Materialien mit unterschiedlichen Materialdicken nachteilig.

Des Weiteren ist in der US 2011/0264001 A1 ein System zur Überwachung der Körpertemperatur eines Patienten beschrieben, bei dem ein gekrümmter Temperatursensor mittels eines lösbaren Sicherheitsstreifens auf der Haut eines Patienten befestigbar und festklebbar ist.

Die WO 2019/063488 A2 offenbart einen Wundverband mit einer Substratschicht als elektrische Verbindung mit Leiterbahnen und einem elektronisches Modul und/oder Sensor. Die Substratschicht mit den Leiterbahnen und der Sensor sind von einer Beschichtungsschicht als Kontaktschicht umgeben, welche im Bereich des Sensors in Richtung zur Wunde ausgestülpt ist.

In der US 10 080 524 B1 ist ein tragbares Thermometerpflaster beschrieben, welches ein flexibles Schaltungssubstrat und eine darunter angeordnete Schicht als Kontaktschicht aufweist, wobei die Kontaktschicht eine Vielzahl von Durchbruchlöchern aufweist, in denen Temperatursensoreinheiten angeordnet sind und dadurch direkt auf der Haut aufliegen.

Die US 2018/256100 A1 beansprucht ein tragbares Vielzweckpflaster mit einem streckbaren und durchlässigen Substrat, welches Durchlässe zur Aufnahme einer Sensoreinheit und von Elektroden aufweist, welche jeweils von einem elektrisch leitfähigen Napf aufweisend hartes Material umgeben sind, welcher direkt auf der Haut aufliegt.

Aufgabe der Erfindung ist es, den Stand der Technik zu verbessern.

Gelöst wird die Aufgabe durch eine Sensorschicht zum Ermitteln von Temperaturprofilen an einer Hautoberfläche mit einer Kontaktschicht zur Hautoberfläche, wobei die Sensorschicht eine untere Lage und eine obere Lage aufweist und auf einer Oberseite der oberen Lage die Kontaktschicht angeordnet ist und die Kontaktschicht über ihre Schichtoberseite in einen Kontakt zur Hautoberfläche stehbar ist, wobei zwischen der unteren Lage und der oberen Lage Leiterbahnen angeordnet sind, welche mit mehreren Temperatursensoren, die auf der Oberseite der oberen Lage angeordnet sind, elektrisch verbunden sind, wobei jeder Temperatursensor mittels einer elektrischen Verbindung zwischen zwei Leiterbahnen verbunden ist, sodass die Sensorschicht flexibel ausgebildet ist und über die Kontaktschicht eine Temperaturdifferenz an der Hautoberfläche ermittelbar ist, wobei die Leiterbahnen durch eine gegenüber Dehnen, Biegen, Strecken und/oder Knicken unempfindliche Leitpaste ausgebildet sind und die Leitpaste zwischen der unteren Lage und der oberen Lage angeordnet ist.

Somit wird eine flexible Sensorschicht zum Erkennen und Beobachten von Temperaturdifferenzen auf der Haut und damit zur Früherkennung von Entzündungen durch Monitoring von einer Hautoberfläche oder mehreren Hautoberflächen und/oder Hautbereichen bereitgestellt. Eine ermittelte negative Temperaturdifferenz an der Position des entsprechenden Temperatursensors deutet beispielsweise auf eine mangelnde Durchblutung des entsprechenden Hautareals hin. Dagegen weist eine positive Temperaturdifferenz bei einem Temperatursensor im Vergleich zu den anderen Temperatursensoren auf eine lokale Temperaturerhöhung und somit auf eine sich anbahnende Entzündung hin. Folglich ist eine sich ausbildende Entzündung auf, unter und/oder in der Haut frühzeitig erkennbar und kann anschließend rechtzeitig behandelt werden, ohne dass Folgeschäden auftreten. Dadurch wird eine Prävention von Entzündungen, Ulcera und Amputationen durch ein hochaufgelöstes Monitoring der Hauttemperatur über eine Hautoberfläche und/oder als Differenz von zwei oder mehreren Hautoberflächen ermöglicht.

Die flexible Sensorschicht dient zum diskontinuierlichen oder kontinuierlichen Messen der Temperatur und somit einem zeitlichen Monitoring der Temperatur bei einer Person mit einer bekannten, bereits festgestellten Erkrankung oder bei einer gefährdeten Person als Indikator und somit als Hinweis auf eine mögliche Erkrankung. Beispielsweise kann eine Temperaturerhöhung auf eine Entzündung hinweisen. Die Deutung als krankhafter Zustand und/oder das Feststellen der Art der entzündungsbildenden Erkrankung unterliegt einem Arzt und fällt nicht in die direkte Verwendung der flexiblen Sensorschicht.

Ein wesentlicher Gedanke der Erfindung liegt darin, dass die Sensorschicht derart ausgebildet ist, dass diese optimal flexibel dreidimensional an eine Hautoberfläche anformbar ist und mittels der Temperatursensoren die gesamte anliegende Hautoberfläche, auch bei Bewegung des Hautareals, überwacht wird, ohne dass durch die Leiterbahnen und/oder die Temperatursensoren ein Druck auf die Hautoberfläche ausgeübt wird. Hierbei sind die, insbesondere flexiblen, Leiterbahnen und die Temperatursensoren auf der mindestens einen Lage der Sensorschicht gerade derart eingebettet, dass diese über die Kontaktschicht keinen vermehrten Druck oder eine andersartige Reizung auf die Hautoberfläche ausüben. Es ist besonders vorteilhaft, dass die gesamte Sensorschicht inklusive der Leiterbahnen und nicht nur die Kontaktschicht flexibel ausgebildet ist.

Insbesondere ist die funktionelle Sensorschicht derart eingerichtet, dass diese ein Messen und Identifizieren von Temperaturdifferenzen zum Auffinden einer Entzündung ermöglicht. Ein Messen von Absolutwerten der Temperatur ist hierbei nicht erforderlich.

Das körpernahe Messen von Temperaturdifferenzen erfolgt durch mindestens eine Schicht. Dabei handelt es sich um die Kontaktschicht der Sensorschicht und/oder durch eine zusätzliche Textilschicht, wie ein Kleidungsstück, welches die Hautoberfläche umgibt. Somit stellt die Kontaktschicht unmittelbar oder mittelbar über das an der Haut getragene Textilstück den Kontakt zur Hautoberfläche dar.

Hierbei ist es besonders vorteilhaft, dass die Sensorschicht mit den Temperatursensoren eine Stärke in einem Bereich von 0,5 mm bis 2,5 mm, bevorzugt von 1,0 mm bis 2,0 mm aufweist.

Dadurch wird zum einen eine gute Wärmeübertragung zwischen der Kontaktschicht und den Temperatursensoren sowie zum anderen ein Vermeiden von Druckreizen durch die Leiterbahnen und/oder Temperatursensoren gewährleistet.

Folgendes Begriffliche sei erläutert:
Eine "Sensorschicht" ist insbesondere ein dreidimensionaler Körper, welcher durch zwei Flächen als Schichtunterseite und Schichtoberseite begrenzt ist, eine geringere Schichtdicke als die Ausdehnung der Flächen der Schichtober- und -unterseite aufweist und einen oder mehrere Messsensoren aufweist. Die Sensorschicht weist zwei Lagen und optional eine Schicht oder mehrere Schichten auf. Erfindungsgemäß weist die Sensorschicht an ihrer Schichtoberfläche eine Kontaktschicht und/oder an ihrer Schichtunterfläche ein Hilfsmittel auf. Die Sensorschicht ist insbesondere flexibel und/oder dehnbar.

Unter einem "Temperaturprofil" wird insbesondere ein Verlauf der Temperatur entlang und/oder über eine Hautoberfläche und/oder über die Zeit an demselben Temperatursensor oder denselben Temperatursensoren und/oder der zugeordneten, derselben Hautstelle oder den zugeordneten, denselben Hautstellen verstanden. Somit wird unter einem Temperaturprofil ein räumliches und/oder zeitliches Temperaturprofil verstanden. Das Temperaturprofil der entsprechenden Hautoberfläche kann beispielsweise mittels einer Smartphone-App online visualisiert werden.

Eine "Hautoberfläche" ist insbesondere die Oberfläche einer Haut, welche einen Körper und/oder ein Körperteil bedeckt. Bei der Hautoberfläche handelt es sich insbesondere um die Oberfläche der äußersten Schicht (Oberhaut, Epidermis) der Haut.

Eine "Kontaktschicht" (auch "Polsterschicht" genannt) ist insbesondere eine Materialschicht mit dünner Schichtdicke und flächiger Ausdehnung, bei der eine Flächenseite im direkten und/oder indirekten Kontakt zur Hautoberfläche steht. Erfindungsgemäß tritt die Kontaktschicht über ihre Schichtoberseite in einen Kontakt zur Hautoberfläche. Eine "Polsterschicht" weist insbesondere ein Material auf, welches eine mechanische Dämpfung, einen Größenausgleich, eine Flächenanpassung und/oder eine Einbettung und/oder Stützung der zur Hautoberfläche zugehörigen Gliedmaße bewirkt. Eine Polsterschicht weist insbesondere eine elastische Federung, Isolierung und/oder Wattierung auf. Als Polsterschicht können insbesondere natürliche oder synthetisch hergestellte Stoffe genutzt werden, wie beispielsweise pflanzliche oder tierische Fasern oder synthetisch hergestellte Schaumstoffe und Schaumvliese sowie andere Kunststoffe und Polymere.

Es ist besonders vorteilhaft, dass die Kontaktschicht, welche im direkten oder indirekten Kontakt mit der Hautoberfläche steht, temperaturisolierend und/oder wärmeisolierend ist. Aufgrund der temperaturisolierenden Eigenschaften der Kontaktschicht wird eine kurzzeitige lokale Temperaturerhöhung an der Hautoberfläche gedämpft und somit eine Fehlinterpretation der Temperaturdaten vermieden. Da sich Entzündungen bekanntermaßen relativ langsam ausbilden, werden dadurch Fehlmaßnahmen und unnötige Behandlungen verhindert. Mithin erfolgt somit eine "Steady State"-Messung.

Als eine "Lage" wird insbesondere eine Schicht eines einzelnen Bogens verstanden. Eine Lage kann insbesondere Papier, Pappe, Folie, Kunststoff und/oder Textil aufweisen. Bei der "unteren Lage" der Sensorschicht handelt es sich um diejenige Lage, welche an der Unterseite der Sensorschicht angeordnet ist und/oder im Kontakt zum Hilfsmittel steht. Bei der "oberen Lage" der Sensorschicht handelt es sich um diejenige Lage, auf welcher die Temperatursensoren sowie die Kontaktschicht angeordnet sind.

Ein "Temperatursensor" ist insbesondere ein elektrisches oder elektronisches Bauelement, welches ein elektrisches Signal als Maß für eine Temperatur und/oder Temperaturverteilung liefert.

Ein Temperatursensor ist insbesondere ein Thermistor, wie beispielsweise ein NTC-Thermistor (Negative Temperature Coefficient Thermistor), welcher einen negativen Temperaturkoeffizienten aufweist und bei hohen Temperaturen den elektrischen Strom besser als bei tiefen Temperaturen leitet. Ebenso kann es sich bei dem Temperatursensor um einen PTC-Thermistor (Positive Temperature Coefficient Thermistor) handeln, welcher einen positiven Temperaturkoeffizienten aufweist und bei tiefen Temperaturen den elektrischen Strom besser als bei hohen Temperaturen leitet. Jeder Temperatursensor an der Oberfläche der oberen Lage der Sensorschicht ist jeweils zwischen zwei Leiterbahnen verbunden. Hierzu ist der Temperatursensor beispielsweise auf die Kontaktfläche, beispielsweise Goldkontaktfläche, jeder Leiterbahn durch Kleben oder Löten kontaktiert. Die Temperatursensoren können jedoch auch mit einer dünnen Lage, beispielsweise einer dünnen Polyurethanschicht, abgedeckt sein und über diese dünne Lage im Kontakt mit der Kontaktschicht stehen.

Eine "Temperaturdifferenz" ist insbesondere der Unterschied in den Temperaturen zweier oder mehrerer Temperaturmessstellen und/oder Temperatursensoren. Eine Temperaturdifferenz eines Temperatursensors oder mehrerer Temperatursensoren zu den anderen Temperatursensoren der Sensorschicht kann insbesondere auf eine Entzündung oder einen andersartigen Defekt der Haut hinweisen.

Eine "Leiterbahn" ist insbesondere eine elektrisch leitende Verbindung mit "zweidimensionalem" Verlauf. Die Leiterbahn verläuft insbesondere in einer Ebene (Leiterbahnebene) und dient insbesondere zur Strom- und/oder Spannungsversorgung, Signalübertragung und/oder Temperaturableitung. Die Leiterbahnen können auch in mehreren Leiterbahnebenen mit dazwischen angeordneten elektrisch isolierenden Ebenen angeordnet werden, wobei beispielsweise eine Verbindung zwischen den einzelnen Leiterbahnebenen mittels vertikaler, elektrisch leitender Verbindungen erfolgt. Eine Leiterbahn weist insbesondere ein elektrisch leitendes Material, beispielsweise Kupfer und/oder eine Kupferlegierung, auf. Erfindungsgemäß sind die Leiterbahnen durch eine Leitpaste, insbesondere Silberleitpaste, ausgebildet. Erfindungsgemäß ist die Leitpaste sandwichmäßig zwischen der unteren Lage und der oberen Lage angeordnet. Die Ausbildung der Leiterbahnen durch Leitpaste ist besonders vorteilhaft, da insbesondere Silberleitpaste unempfindlich gegenüber Dehnen, Biegen, Strecken und/oder Knicken ist.

Erfindungsgemäß weist die Sensorschicht eine untere Lage und eine obere Lage auf und auf der oberen Lage ist die Kontaktschicht angeordnet, wobei zwischen der unteren Lage und der oberen Lage Leiterbahnen angeordnet sind, welche mit mehreren Temperatursensoren auf einer Oberseite der oberen Lage elektrisch verbunden sind.

Dadurch, dass die Leiterbahnen zwischen der unteren Lage und der oberen Lage der Sensorschicht eingebettet sind, können zum einen die Leiterbahnen in einfacher Weise zwischen die Oberseite der unteren Lage und der Unterseite der oberen Lage eingebracht werden. Zum anderen sind die Leiterbahnen zwischen den beiden Lagen optimal einbettet, sodass diese über die Kontaktschicht keinen vermehrten Druck oder eine andersartige Reizung auf die Hautoberfläche ausüben.

In einer weiteren Ausführungsform der Sensorschicht sind die Leiterbahnen gleichmäßig beabstandet, mäanderförmig und/oder aderförmig ausgebildet.

Durch eine gleichmäßige Beabstandung der Leiterbahnen, beispielsweise durch parallele Ausführung oder als Quadratnetz, sind diese weitgehend optimal flächengleich zu einer darüber oder darunter angeordneten Schicht ausführbar.

Es ist besonders vorteilhaft, wenn die Leiterbahnen aderförmig und/oder mäanderförmig ausgebildet sind, da dadurch die Flexibilität der Sensorschicht weiter verbessert wird. Somit sind die Leiterbahnen dynamisch beweglich ausgebildet. Folglich wird durch Dehnen, Biegen, Strecken und/oder Knicken der Sensorschicht ein Brechen der Leiterbahnen und somit der elektrischen Verbindungen verhindert sowie ein optimales Anformen an die Hautoberfläche ermöglicht und eine Druckbelastung der Haut vermieden. Hierzu sind die Leiterbahnen technisch insbesondere derart ausgebildet, dass die Leiterbahnen dynamischen Kräften, wie Dehnung und Stauchung, über einen längeren Zeitraum, insbesondere mindestens im Messzeitraum, standhalten.

Bevorzugt erstrecken sich die mäanderförmig und/oder aderförmig Leiterbahnen in der horizontalen Ebene von einer elektronischen Schaltung, insbesondere Platine, aus fingerförmig in die Peripherie bis zu den Temperatursensoren. Dadurch wird eine erhöhte Flexibilität erzielt und folglich eine bessere dreidimensionale Anpassung an eine gewünschte Form, wie beispielsweise an ein Hilfsmittel oder einen Träger der Sensorschicht erreicht.

Unter "mäanderförmig" wird insbesondere ein Verlauf in Form von Schlingen, Windungen und/oder Schleifen, welche aufeinanderfolgen, verstanden.

Unter "aderförmig" wird insbesondere verstanden, dass die Leiterbahnen eine Form von sich verzweigenden Adern aufweisen.

Um die Sensorschicht flexibel und/oder dehnbar auszugestalten, weist die Sensorschicht einen flexiblen Kunststoff, insbesondere Polyurethan, auf.

Es ist besonders vorteilhaft, dass die Sensorschicht aufgrund ihres Materials, insbesondere flexibles Polyurethan (PU) oder einem anderen flexiblen Kunststoff, und/oder aufgrund der eingebetteten dynamischen Leiterbahnen zwischen der unteren und der oberen Lage flexibel ausgestaltet ist. Somit kann die Sensorschicht eine Dehnbarkeit bis zu 30 % ihrer Abmessung im unbeanspruchten Zustand aufweisen.

Es ist besonders vorteilhaft, dass die Sensorschicht einen vernetzbaren Kunststoff aufweist, sodass die Flexibilität des Kunststoffes bedarfsgerecht eingestellt werden kann. Beispielsweise können die Eigenschaften von Polyurethan über den Vernetzungsgrad und/oder die eingesetzten Komponenten (insbesondere Isocyanat- oder OH-Komponenten) in einem weiten Bereich variiert werden, sodass Duroplaste, Thermoplaste oder Elastomere vorliegen.

"Polyurethan" ist insbesondere ein Kunststoff oder Kunstharz, welcher aus der Polyadditionsreaktion von Dialkoholen und/oder Polyolen mit Polyisocyanaten entsteht. Polyurethan kann insbesondere als Weich- oder Hartschaumstoff oder als textiler elastischer Faserstoff vorliegen.

In einer weiteren Ausführungsform der Sensorschicht weist jeder Temperatursensor eine maximale Abmessung von ≤ 2 mm**,** insbesondere ≤ 1,8 mm, bevorzugt ≤ 1,5 mm, auf.

Aufgrund der geringen Höhe, Breite und Länge jedes Temperatursensors nimmt jeder Temperatursensor auf der Oberseite der oberen Lage nur einen sehr geringen Platz ein und führt aufgrund der darüber angeordneten Kontaktschicht (Polsterschicht) nicht zu einer Druckbelastung der Hautoberfläche.

Um die gesamte, relevante Hautoberfläche messtechnisch zu erfassen, weist jeder Temperatursensor einen Messradius in einem Bereich von 2,5 cm bis 1,5 cm, bevorzugt von 2,3 cm bis 1,7 cm, auf.

Bevorzugt sind die Temperatursensoren über die Oberfläche der oberen Lage der Sensorschicht gleichmäßig und/oder flächendeckend angeordnet, sodass aufgrund der Messradien der Temperatursensoren die gesamte Hautoberfläche, welche mit der Kontaktschicht in Kontakt steht, sensorisch und/oder messtechnisch erfasst wird. Zudem ist der Messradius jedes Temperatursensors derart sensibel ausgelegt, dass dieser auch mehrere Schichten (wie Textilschichten) durchdringen kann.

Als "Messradius" wird insbesondere ein Abstand zwischen dem Mittelpunkt eines Temperatursensors und einer darum angeordneten Kreislinie verstanden, in dem der Temperatursensor eine Temperatur misst. Der Messradius endet insbesondere auf der Kugelaußenfläche einer dreidimensionalen Kugel um den Mittelpunkt des Messsensors.

Um eine Fehlinterpretation der Temperaturdaten bei einer kurzzeitigen lokalen Temperaturerhöhung zu vermeiden, ist die Kontaktschicht temperaturisolierend, sodass eine kurzzeitige Temperaturerhöhung an der Hautoberfläche gedämpft ermittelt wird.

Durch die Dämpfung einer ermittelten Temperaturerhöhung mittels der Kontaktschicht wird eine entsprechende Temperaturdifferenz der Haut nicht sofort als Überschreitung eines vorgegebenen Schwellenwertes interpretiert und somit ein Fehlalarm und unnötige Behandlungsschritte vermieden.

Unter "temperaturisolierend" wird insbesondere verstanden, dass die Kontaktschicht eine derartige Materialeigenschaft aufweist, dass eine Reduktion des Durchgangs von Wärme oder Kälte durch die Kontaktschicht erfolgt. Dadurch wird die Temperatur an der Hautoberfläche insbesondere gedämpft durch die Kontaktschicht an den unterhalb der Kontaktschicht angeordneten Temperatursensoren erfasst.

Um als Träger für die Sensorschicht zu dienen und diese optimal an die Hautoberfläche anzuformen, ist die Sensorschicht über eine Unterseite der mindestens einen Lage oder der unteren Lage mit einem Hilfsmittel verbunden.

Zum einen dient das Hilfsmittel zum Fixieren der Sensorschicht am Körper, insbesondere über Formschluss. Dadurch schmiegen sich das Hilfsmittel und somit die Sensorschicht aufgrund deren Elastizitäten und/oder des Zuschnittes dreidimensional an die Form des zu messenden Körperbereiches an. Damit wird gewährleistet, dass auch beim erneuten Anziehen, Anlegen und/oder Benutzen des Hilfsmittels jeder Temperatursensor wiederholbar an seiner vorgegebenen Position zur Hautoberfläche sitzt.

Ein "Hilfsmittel" ist insbesondere an der Unterseite der unteren Lage der Sensorschicht angeordnet. Bei einem Hilfsmittel handelt es sich beispielsweise um eine Sohle, Bandage, Sitzfläche eines Rollstuhls, Laken für ein Pflegebett, Liner in einem Prothesenschaft oder einen ähnlichen Gegenstand, welcher direkt oder indirekt mit einer Hautoberfläche in Berührung kommt. Neben einer Bandage, bei der eine Bewegung des bandagierten Körperteils möglich ist, kann es sich bei einem Hilfsmittel auch um eine Orthese zur Ruhigstellung des Körperteils handeln. Ein Hilfsmittel ist selbstverständlich nicht auf medizinische Anwendungen beschränkt. Ein Hilfsmittel ist insbesondere auch jede Art von Gebrauchsgegenstand, welcher mit einer Körperoberfläche in Kontakt kommen kann. Beispielsweise handelt es sich bei einem Hilfsmittel auch um eine Sohle in einem Sportschuh oder um eine Band für ein Fitnessarmband.

In einer weiteren Ausführungsform der Sensorschicht sind die Leiterbahnen elektrisch mit einer elektronischen Schaltung, insbesondere Platine, im und/oder am Hilfsmittel verbunden.

Somit dient das Hilfsmittel gleichzeitig auch als Halterung einer der Sensorschicht zugeordneten Temperatursensorik, insbesondere einer elektronischen Schaltung. Durch die Integration einer elektronischen Schaltung, beispielsweise Platine, in und/oder am Hilfsmittel, ist die üblicherweise unflexible elektronische Schaltung bevorzugt außerhalb des druckbelasteten Bereiches des Körpers angeordnet, sodass ebenfalls kein Druck von der elektronischen Schaltung auf die Hautoberfläche ausgeübt wird.

Eine "elektronische Schaltung" ist insbesondere ein Zusammenschluss von elektrischen und/oder elektronischen Bauelementen zu einer funktionierenden Anordnung. Bei einer elektronischen Schaltung handelt es sich beispielsweise um eine Leiterplatte oder Platine. Die elektronische Schaltung weist verschiedene Bauteile, wie beispielsweise eine Energieversorgung, Mikrocontroller, Datenspeicher, Echtzeituhr, Bluetooth-Modul, Multiplexer und ähnliches, auf.

Um eine telemedizinische Datenerfassung und -überwachung zu ermöglichen, weist die elektronische Schaltung ein Kommunikationsmodul zum Übertragen von Daten an ein Kontrollgerät auf.

Mittels eines Kommunikationsmoduls, beispielsweise eines Bluetooth-Moduls, können die Temperaturdaten an ein Kontrollgerät drahtlos übertragen werden. Somit ist ein kontinuierliches Monitoring von prädestinierten Hautstellen, beispielsweise via einer Smartphone-App, möglich. Auch kann mittels der Smartphone-App eine Online-Visualisierung und Auswertung der Temperaturdifferenzen eines entsprechenden Hautareals erfolgen. Vorteilhaft kann bei einem überschwelligen Messwert als Hinweis auf ein pathologisches Ereignis der Haut eine Alarmfunktion mittels der App aktiviert werden. Zudem werden dadurch eine klinische und/oder präventive Überwachung und eine aktive, stetige Kontrolle von Therapieansätzen durch telemedizinische Datenerfassung ermöglicht. Hierbei ist zur telemedizinischen Therapiekontrolle ein Datentransfer auf eine übergeordnete Datenbank vorteilhaft.

Unter einem "Kommunikationsmodul" wird insbesondere eine elektronische Baugruppe verstanden, welche Daten übernimmt und beispielsweise an ein externes Gerät, wie ein Kontrollgerät, überträgt. Bei einem Kommunikationsmodul handelt es sich insbesondere um eine Transceiver-Baugruppe sowie dazugehörige Komponenten zur Ansteuerung (Mikrocontroller). Die Baugruppen des Kommunikationsmoduls können beispielsweise als Steckkarten auf einer Platine ausgeführt sein. Insbesondere übernimmt das Kommunikationsmodul die Sende- und Empfangsprotokolle, die Verschlüsselung, Verwaltung der Daten sowie die Ansteuerung zur Übertragung. Ein Kommunikationsmodul überträgt insbesondere drahtgebunden oder drahtlos. Das Kommunikationsmodul weist beispielsweise eine Bluetooth-Schnittstelle, ein Funkmodul, einen RFID-Transponder oder ein anderes Übertragungsgerät auf.

Ein "Kontrollgerät" ist insbesondere ein Computer, Tablet, Smartphone oder ein sonstiges Überwachungsgerät, welches die Daten vom Kommunikationsmodul empfängt und weiterverarbeitet. Das Kontrollgerät dient insbesondere auch zur Ausgabe eines Alarms beim Überschreiten eines Schwellenwertes, zur Eigenkontrolle durch einen Patienten sowie zur telemedizinischen Therapiekontrolle und -überwachung.

Es ist besonders vorteilhaft, wenn die elektronische Schaltung einen Aktivierungssensor, wie beispielsweise einen sechsachsigen Beschleunigungssensor auf der starren Platine aufweist. Über den Beschleunigungssensor werden bei einer Bewegung die Temperatursensoren aktiviert und gehen dadurch in einen Messmodus über. Somit wird nicht dauerhaft mittels der Temperatursensoren gemessen, sondern nur beim Bewegen und/oder Tragen des Hilfsmittels. Dadurch wird der Energiebedarf vermindert und programmierbare Aktionen bei Beschleunigungsmustern werden ermöglicht. Beispielsweise erfolgt im Falle einer Sohle als Hilfsmittel das Versenden der Daten durch das Kommunikationsmodul automatisch, wenn vom Beschleunigungssensor detektiert wird, dass die Sohle auf dem Kopf gedreht wird und somit die Messungen mit der Sohle abgeschlossen sind.

In einem weiteren Aspekt der Erfindung wird die Aufgabe gelöst durch ein Hilfsmittel zum Anlegen an eine Hautoberfläche, wobei das Hilfsmittel eine zuvor beschriebene Sensorschicht aufweist.

Somit kann mittels des Hilfsmittels reproduzierbar und wiederholbar die Sensorschicht optimal an eine Hautoberfläche angelegt werden. Vor allem kann je nach Lage der betroffenen Hautoberfläche das optimale Hilfsmittel zum Ermitteln eines Temperaturprofils der entsprechenden Hautoberfläche mittels der verbundenen Sensorschicht gewählt werden.

Ein Hilfsmittel wurde vorliegend bereits definiert. Bei dem Hilfsmittel kann es sich beispielsweise um einen Gegenstand handeln, welcher direkt an und/oder um ein Körperteil an- oder umgelegt wird, wie eine Bandage, oder um einen Gegenstand auf dem ein Mensch sitzt oder liegt, wie die Sitzfläche eines Rollstuhls oder das Laken in einem Pflegebett.

In einem zusätzlichen Aspekt der Erfindung wird die Aufgabe gelöst durch ein Verfahren zum Herstellen eines Hilfsmittels gemäß Anspruch 11.

Beispielsweise werden zum Herstellen einer Schuhsohle mit Sensorschicht als Hilfsmittel zunächst Leiterbahnen in die Lage eingebracht. Anschließend werden mehrere Temperatursensoren entsprechend des zu überwachenden Hautbereiches eines Fußes in der Lage derart angeordnet, dass jeder Temperatursensor an der Oberseite der Lage angrenzt, wobei jeder Temperatursensor zwischen zwei Leiterbahnen geklebt oder eingelötet wird. Anschließend wird die Lage mit den Leiterbahnen und den Temperatursensoren als vorgefertigte Sensorschicht mit der Unterseite auf einen Sohlenrohling geklebt. Auf der Oberseite der Lage, dort, wo die Temperatursensoren angeordnet sind, wird eine Polsterschicht als Kontaktschicht aufgeklebt. Anschließend erfolgt eine Formgebung des Sohlenrohlings mit der eingebetteten Sensorschicht durch Schneiden, Stanzen und/oder Abschleifen von Überständen.

Im Falle, dass die Sensorschicht eine untere Lage und eine ober Lage aufweist, können folgende Schritte durchgeführt werden:
- Aufbringen von Leiterbahnen auf eine Oberseite einer unteren Lage,
- Auflegen einer oberen Lage auf die untere Lage mit den Leiterbahnen,
- Anordnen von mehreren Temperatursensoren auf einer Oberseite der oberen Lage und verbinden jedes Temperatursensors zwischen zwei Leiterbahnen,
- Aufbringen einer Kontaktschicht auf die Oberseite der oberen Lage mit den Temperatursensoren, sodass eine Sensorschicht ausgebildet ist, und
- Aufbringen der Unterseite der unteren Lage auf einen Hilfsmittelrohling oder auf ein Hilfsmittel und/oder Formgebung des Hilfsmittelrohlings mit der verbundenen Sensorschicht, sodass ein Hilfsmittel vorliegt.

In einem weiteren Aspekt der Erfindung wird die Aufgabe gelöst durch ein Verfahren zum Ermitteln einer relativen Temperaturdifferenz an einer Oberfläche gemäß Anspruch 12**.**

Somit wird ein Verfahren zum Ermitteln einer relativen Temperaturdifferenz und zum frühzeitigen Erkennen von Substanzdefekten an der Hautoberfläche bereitgestellt. Es ist besonders vorteilhaft, dass mittels des erfindungsgemäßen Verfahrens kontinuierlich auch langfristig eine Überwachung der Hautoberfläche ermöglicht wird, ohne dass dadurch aufgrund der Messsensorik eine Druckbelastung der Haut und somit eine Verschlechterung von Substanzdefekten auftritt.

Bevorzugt wird nach Messen von Temperaturen mittels der mehreren Temperatursensoren ein Vergleich der aufgenommenen Messwerte mit jeweils Messwerten benachbarter Temperatursensoren und/oder mit früher aufgenommenen Messwerten durchgeführt.

Im Falle von zwei parallel verwendeten Hilfsmittels, beispielsweise einer rechten und linken Sohle, werden die Messwerte des einen Hilfsmittels mit den Messwerten des komplementären anderen Hilfsmittel verglichen. Hierbei ist die Verknüpfung der ermittelten Werte aus verschiedenen Hilfsmitteln über ein Kommunikationsmodul möglich, wobei das Kommunikationsmodul insbesondere Daten empfangen kann, sobald bereits eines der Hilfsmittel Betriebsbereitschaft an das Kommunikationsmodul meldet.

Somit werden vorteilhaft mindestens zwei Abgleiche oder auch mehrere Abgleiche durchgeführt, wobei die Abgleiche jeweils aktuelle, aufgenommene Messwerte derselben und/oder einer komplementären Sensorsicht oder Hilfsmittels betreffen und/oder in der Vergangenheit aufgenommen wurden. Bevorzugt werden mindestens zwei Abgleiche durchgeführt, bevor eine Temperaturdifferenz als schädlich ausgegeben wird.

In einem zusätzlichen Schritt des Verfahrens wird eine Steuerung des Messens über einen sechsachsigen Beschleunigungssensor durchgeführt. Der Beschleunigungssensor aktiviert die Temperatursensoren und versetzt diese dadurch in den Messmodus. Folglich wird nur beim Verwenden und/oder beim Tragen des Hilfsmittels mittels der Temperatursensoren gemessen. Dadurch wird der Energiebedarf der Sensorschicht und/oder des Hilfsmittels minimiert.

Zudem können andere programmierbare Aktionen bei Beschleunigungsmustern vorgegeben werden, wie beispielsweise das Initiieren einer Datenübertragung.

Im Weiteren wird die Erfindung anhand von Ausführungsbeispielen näher erläutert. Es zeigen
- Figur 1: eine stark schematische, nicht maßstabsgerechte Schnittdarstellung einer Sensorschicht mit einem verbundenen Hilfsmittelrohling und einer Polsterschicht, und
- Figur 2: eine stark schematische Draufsicht auf eine untere PU-Lage der Sensorschicht mit Leiterbahnen.

Eine Sensorschicht 101 weist eine untere PU-Lage 107 und eine obere PU-Lage 105 auf. Zwischen der unteren PU-Lage 107 und der oberen PU-Lage 105 sind Leiterbahnen 109 angeordnet. Die Leiterbahnen 109 sind anpassungsfähig und ader- und mäanderförmig über eine Oberfläche der unteren PU-Lage 107 verteilt und sind seitlich zwischen der unteren PU-Lage 107 und der oberen PU-Lage 105 als elektrische Verbindung 119 heraus zur elektrischen Kontaktierung einer Platine 113 geführt, welche im Hilfsmittelrohling 115 angeordnet ist.

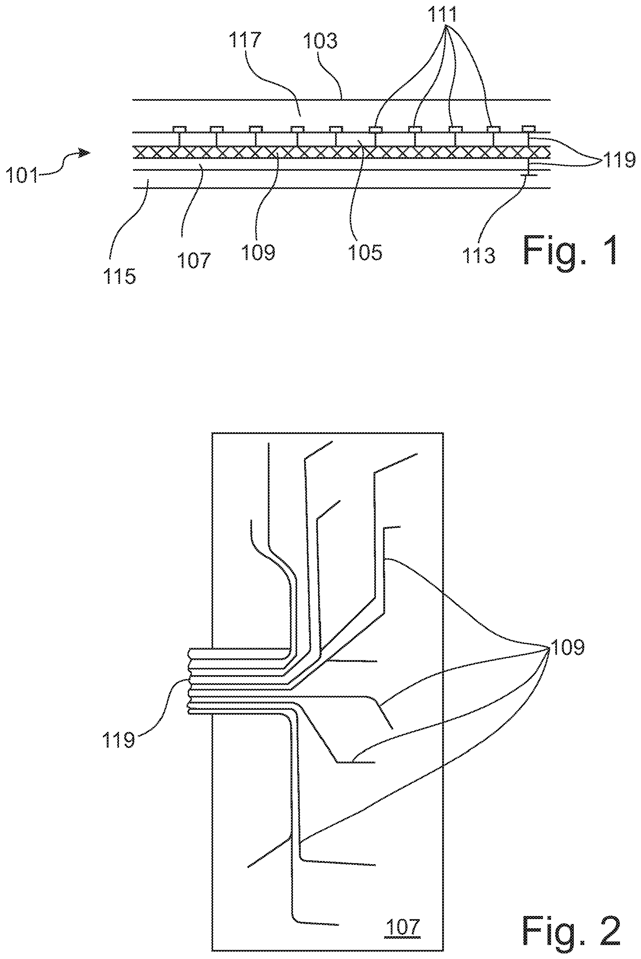

Auf der oberen PU-Lage 105 sind mehrere Temperatursensoren 111 angeordnet. Jeder Temperatursensor 111 ist mittels einer elektrischen Verbindung 119 zwischen zwei Leiterbahnen 109 verbunden. Die Temperatursensoren 111 weisen jeweils eine Höhe von 1,5 mm (in Figur 1 nicht maßstabsgerecht dargestellt) und einen Messradius von 2,0 cm auf. Auf die Oberseite der oberen PU-Lage 105 mit den Temperatursensoren 111 ist eine Polsterschicht 117 mit einer Kontaktfläche 103 geklebt.

Die Sensorschicht 101 mit der Polsterschicht 117 und dem unten verklebten Hilfsmittelrohling 115 wird zugeschnitten und als Pad in einer Kniebandage eingesetzt. Die Kniebandage wird an ein frisch operiertes Knie eines Patienten angebracht, wobei sich die Sensorschicht 101 aufgrund der PU-Lagen 105, 107 und der flexiblen, mäanderförmigen Leiterbahnen 109 optimal an das Knie anformen lässt, ohne Druck auf das frisch operierte Knie aufzubringen. Somit steht die Kontaktfläche 103 direkt in Kontakt zur Hautoberfläche des Knies.

Mittels der Temperatursensoren 111 wird kontinuierlich die jeweilige Temperatur gemessen und über die Platine 113 via Bluetooth an ein nicht gezeigtes zentrales Kontrollgerät übermittelt. Im Kontrollgerät werden die Temperaturdifferenzen zwischen den einzelnen Temperatursensoren 111 bestimmt und gespeichert. Über 36 h wird eine zunehmende lokale Temperaturerhöhung bei einem der Temperatursensoren 111 festgestellt, und bei Überschreiten eines festgelegten Schwellenwertes ein Alarm ausgelöst. Bei einer medizinischen Kontrolle wird entsprechend eine Entzündung an der Operationsnaht relativ zu Position des einen Temperatursensors 111 festgestellt und daraufhin zeitnah durch einen Arzt behandelt, ohne dass ein Folgeschaden auftritt.

In einer nicht-erfindungsgemäßen Alternative weist eine nicht gezeigte Sensorschicht eine nicht gezeigte einlagige PU-Schicht auf. Die Leiterbahnen 109 und die Temperatursensoren 111 sind in dieser PU-Schicht eingebettet, wobei die Temperatursensoren 111 mit ihrer Oberseite an die Oberseite der PU-Schicht bündig abschließen. Jeder Temperatursensor 111 ist zwischen zwei Leiterbahnen 109 elektrisch verbunden. Auf die Oberseite der einlagigen PU-Schicht mit den Temperatursensoren 111 ist eine Polsterschicht 117 mit einer Kontaktfläche 103 geklebt. Mit ihrer Unterseite ist die einlagige PU-Schicht auf einem Hilfsmittelrohling 115 geklebt. Die Temperaturmessungen mit dieser alternativen Sensorschicht erfolgt entsprechend wie oben beschrieben.

### Bezugszeichenliste

- 101: Sensorschicht
- 103: Kontaktfläche
- 105: obere PU-Lage
- 107: untere PU-Lage
- 109: Leiterbahnen
- 111: Temperatursensor
- 113: Platine
- 115: Hilfsmittelrohling
- 117: Polsterschicht
- 119: elektrische Verbindung

## Patentansprüche

1. Sensorschicht (101) zum Ermitteln von Temperaturprofilen an einer Hautoberfläche mit einer Kontaktschicht (117) zur Hautoberfläche, wobei die Sensorschicht (101) eine untere Lage (107) und eine obere Lage (105) aufweist und auf einer Oberseite der oberen Lage (105) die Kontaktschicht (117) angeordnet ist und die Kontaktschicht (117) über ihre Schichtoberseite in einen Kontakt zur Hautoberfläche stehbar ist, wobei zwischen der unteren Lage (107) und der oberen Lage (105) Leiterbahnen (109) angeordnet sind, welche mit mehreren Temperatursensoren (111), die auf der Oberseite der oberen Lage (105 angeordnet sind, elektrisch verbunden sind, wobei jeder Temperatursensor (111) mittels einer elektrischen Verbindung (119) zwischen zwei Leiterbahnen (109) verbunden ist, sodass die Sensorschicht (101) flexibel ausgebildet ist und über die Kontaktschicht (117) eine Temperaturdifferenz an der Hautoberfläche ermittelbar ist, wobei die Leiterbahnen (109) durch eine gegenüber Dehnen, Biegen, Strecken und/oder Knicken unempfindliche Leitpaste ausgebildet sind und die Leitpaste zwischen der unteren Lage (107) und der oberen Lage (105) angeordnet ist.

2. Sensorschicht (101) nach Anspruch 1, **dadurch gekennzeichnet, dass** die Leiterbahnen (109) gleichmäßig beabstandet, mäanderförmig und/oder aderförmig ausgebildet sind.

3. Sensorschicht (101) nach einem der vorherigen Ansprüche, **dadurch gekennzeichnet, dass** die Sensorschicht (101) einen flexiblen Kunststoff, insbesondere Polyurethan, aufweist.

4. Sensorschicht (101) nach einem der vorherigen Ansprüche, **dadurch gekennzeichnet, dass** jeder Temperatursensor (111) eine maximale Abmessung von ≤ 2 mm, insbesondere ≤ 1,8 mm, bevorzugt ≤ 1,5 mm, aufweist.

5. Sensorschicht (101) nach einem der vorherigen Ansprüche, **dadurch gekennzeichnet, dass** jeder Temperatursensor (111) einen Messradius in einem Bereich von 2,5 cm bis 1,5 cm, bevorzugt von 2,3 cm bis 1,7 cm, aufweist.

6. Sensorschicht (101) nach einem der vorherigen Ansprüche, **dadurch gekennzeichnet, dass** die Kontaktschicht (103) temperaturisolierend ist, sodass eine kurzzeitige Temperaturerhöhung an der Hautoberfläche gedämpft ermittelt wird.

7. Sensorschicht (101) nach einem der vorherigen Ansprüche, **dadurch gekennzeichnet, dass** die Sensorschicht (101) über eine Unterseite der unteren Lage (107) mit einem Hilfsmittel verbunden ist.

8. Sensorschicht (101) nach einem der vorherigen Ansprüche, **dadurch gekennzeichnet, dass** die Leiterbahnen elektrisch mit einer elektronischen Schaltung, insbesondere Platine (113), im und/oder am Hilfsmittel verbunden sind.

9. Sensorschicht (101) nach einem der vorherigen Ansprüche, **dadurch gekennzeichnet, dass** die elektronische Schaltung ein Kommunikationsmodul zum Übertragen von Daten an ein Kontrollgerät aufweist.

10. Hilfsmittel zum Anlegen an eine Hautoberfläche, **dadurch gekennzeichnet, dass** das Hilfsmittel eine Sensorschicht nach einem der Ansprüche 1 bis 9 aufweist.

11. Verfahren zum Herstellen eines Hilfsmittels, wobei das Hilfsmittel an einer Hautoberfläche anlegbar ist, mit folgenden Schritten:
- Aufbringen von Leiterbahnen (109) ausgebildet durch eine gegenüber Dehnen, Biegen, Strecken und/oder Knicken unempfindliche Leitpaste auf eine Oberseite einer unteren Lage (107),
- Auflegen einer oberen Lage (105) auf die untere Lage (107) mit den Leiterbahnen (109), sodass die Leitpaste zwischen der unteren Lage (107) und der oberen Lage (105) angeordnet ist,
- Anordnen von mehreren Temperatursensoren (111) auf einer Oberseite der oberen Lage (105) und Verbinden jedes Temperatursensors (111) zwischen zwei Leiterbahnen (109) mittels einer elektrischen Verbindung (119),
- Aufbringen einer Kontaktschicht (103) auf die Oberseite der oberen Lage (105) mit den Temperatursensoren (111), sodass eine Sensorschicht (101) ausgebildet ist, und
- Aufbringen einer Unterseite der unteren Lage (107) auf einen Hilfsmittelrohling (115) oder auf ein Hilfsmittel und/oder Formgeben des Hilfsmittelrohlings mit der verbundenen Sensorschicht (101), sodass ein Hilfsmittel vorliegt.

12. Verfahren zum Ermitteln einer relativen Temperaturdifferenz an einer Hautoberfläche, wobei eine Sensorschicht (101) nach einem der Ansprüche 1 bis 9 oder einem Hilfsmittel nach Anspruch 10 verwendet wird, mit folgenden Schritten:
- Anformen der Sensorschicht (101) an die Hautoberfläche, sodass die Kontaktschicht (117) der Sensorschicht (101) in einem Kontakt zur Hautoberfläche oder zu einer die Hautoberfläche umgebenden Textilfläche steht,
- Messen von Temperaturen mittels der mehreren Temperatursensoren (111) in der Sensorschicht (101),
- Ermitteln der relativen Temperaturdifferenz zwischen den mehreren Temperatursensoren (111), sodass anhand einer lokalen Temperaturerhöhung eine Entzündung der Hautoberfläche frühzeitig erkennbar ist.

## Claims

1. Sensor layer (101) for determining temperature profiles on a skin surface, comprising a contact layer (117) with the skin surface, wherein the sensor layer (101) comprises a lower ply (107) and an upper ply (105) and the contact layer (117) is arranged on a top side of the upper ply (105), and the contact layer (117) can be in contact with the skin surface via its layer top side, wherein conductor tracks (109) are arranged between the lower ply (107) and the upper ply (105), which conductor tracks are electrically connected to a plurality of temperature sensors (111) arranged on the top side of the upper ply (105), wherein each temperature sensor (111) is connected between two conductor tracks (109) by means of an electrical connection (119) such that the sensor layer (101) is formed to be flexible and a temperature difference on the skin surface can be determined via the contact layer (117), wherein the conductor tracks (109) are formed by a conductive paste that is insensitive to stretching, bending, elongation and/or buckling and the conductive paste is arranged between the lower ply (107) and the upper ply (105).

2. Sensor layer (101) according to claim 1, **characterised in that** the conductor tracks (109) are formed so as to be evenly spaced, meandering and/or vein-like.

3. Sensor layer (101) according to any of the preceding claims, **characterised in that** the sensor layer (101) comprises a flexible plastics material, in particular polyurethane.

4. Sensor layer (101) according to any of the preceding claims, **characterised in that** each temperature sensor (111) has a maximum dimension of < 2 mm, in particular < 1.8 mm, preferably < 1.5 mm.

5. Sensor layer (101) according to any of the preceding claims, **characterised in that** each temperature sensor (111) has a measuring radius in a range of 2.5 cm to 1.5 cm, preferably 2.3 cm to 1.7 cm.

6. Sensor layer (101) according to any of the preceding claims, **characterised in that** the contact layer (103) is thermally insulating such that a brief temperature increase on the skin surface is determined in an attenuated manner.

7. Sensor layer (101) according to any of the preceding claims, **characterised in that** the sensor layer (101) is connected to an aid via a bottom side of the lower ply (107).

8. Sensor layer (101) according to any of the preceding claims, **characterised in that** the conductor tracks are electrically connected to an electronic circuit, in particular a printed circuit board (113), in and/or on the aid.

9. Sensor layer (101) according to any of the preceding claims, **characterised in that** the electronic circuit comprises a communication module for transmitting data to a controller.

10. Aid for application to a skin surface, **characterised in that** the aid comprises a sensor layer according to any of claims 1 to 9.

11. Method for producing an aid, wherein the aid can be applied to a skin surface, comprising the following steps:
- depositing conductor tracks (109) formed by a conductive paste that is insensitive to stretching, bending, elongation and/or buckling to a top side of a lower ply (107),
- applying an upper ply (105) to the lower ply (107) comprising the conductor tracks (109), such that the conductive paste is arranged between the lower ply (107) and the upper ply (105),
- arranging a plurality of temperature sensors (111) on a top side of the upper ply (105) and connecting each temperature sensor (111) between two conductor tracks (109) by means of an electrical connection (119),
- depositing a contact layer (103) on the top side of the upper ply (105) comprising the temperature sensors (111), such that a sensor layer (101) is formed, and
- depositing a bottom side of the lower ply (107) on an aid preform (115) or on an aid and/or shaping the aid preform comprising the connected sensor layer (101), such that an aid is provided.

12. Method for determining a relative temperature difference on a skin surface, wherein a sensor layer (101) according to any of claims 1 to 9 or an aid according to claim 10 is used, comprising the following steps:
- fitting the sensor layer (101) to the skin surface such that the contact layer (117) of the sensor layer (101) is in contact with the skin surface or with a textile surface surrounding the skin surface,
- measuring temperatures by means of the plurality of temperature sensors (111) in the sensor layer (101),
- determining a relative temperature difference between the plurality of temperature sensors (111) such that an inflammation of the skin surface can be identified at an early stage on the basis of a local temperature increase.

## Revendications

1. Couche de capteur (101) destinée à déterminer des profils de température sur une surface de peau avec une couche de contact (117) avec la surface de peau, dans laquelle la couche de capteur (101) présente une strate inférieure (107) et une strate supérieure (105) et la couche de contact (117) est disposée sur un côté supérieur de la strate supérieure (105) et la couche de contact (117) peut se trouver en contact avec la surface de peau par l'intermédiaire de son côté supérieur de couche, dans laquelle des pistes conductrices (109) sont disposées entre la strate inférieure (107) et la strate supérieure (105), lesquelles sont raccordées électriquement à plusieurs capteurs de température (111), qui sont disposés sur le côté supérieur de la strate supérieure (105), dans laquelle chaque capteur de température (111) est raccordé entre deux pistes conductrices (109) au moyen d'un raccordement électrique (119) si bien que la couche de capteur (101) est réalisée de manière flexible et une différence de température sur la surface de peau peut être déterminée par l'intermédiaire de la couche de contact (117), dans laquelle les pistes conductrices (109) sont réalisées par une pâte conductrice insensible par rapport à l'allongement, à la flexion, à l'étirage et/ou au pliage et la pâte conductrice est disposée entre la strate inférieure (107) et la strate supérieure (105).

2. Couche de capteur (101) selon la revendication 1, **caractérisée en ce que** les pistes conductrices (109) sont réalisées avec un espacement homogène, avec une forme sinueuse et/ou avec une forme filaire.

3. Couche de capteur (101) selon l'une quelconque des revendications précédentes, **caractérisée en ce que** la couche de capteur (101) présente une matière plastique flexible, en particulier du polyuréthane.

4. Couche de capteur (101) selon l'une quelconque des revendications précédentes, **caractérisée en ce que** chaque capteur de température (111) présente une dimension maximale ≤ 2 mm, en particulier ≤ 1,8 mm, de manière préférée ≤ 1,5 mm.

5. Couche de capteur (101) selon l'une quelconque des revendications précédentes, **caractérisée en ce que** chaque capteur de température (111) présente un rayon de mesure dans une plage de 2,5 cm à 1,5 cm, de manière préférée de 2,3 cm à 1,7 cm.

6. Couche de capteur (101) selon l'une quelconque des revendications précédentes, **caractérisée en ce que** la couche de contact (103) est thermoisolante si bien qu'une brève augmentation de température sur la surface de peau est déterminée de manière atténuée.

7. Couche de capteur (101) selon l'une quelconque des revendications précédentes, **caractérisée en ce que** la couche de capteur (101) est raccordée à un moyen auxiliaire par l'intermédiaire d'un côté inférieur de la strate inférieure (107).

8. Couche de capteur (101) selon l'une quelconque des revendications précédentes, **caractérisée en ce que** les pistes conductrices sont raccordées électriquement à un circuit électronique, en particulier une platine (113), dans et/ou sur le moyen auxiliaire.

9. Couche de capteur (101) selon l'une quelconque des revendications précédentes, **caractérisée en ce que** le circuit électronique présente un module de communication destiné à transmettre des données à un appareil de contrôle.

10. Moyen auxiliaire destiné à être placé sur une surface de peau, **caractérisé en ce que** le moyen auxiliaire présente une couche de capteur selon l'une quelconque des revendications 1 à 9.

11. Procédé destiné à fabriquer un moyen auxiliaire, dans lequel le moyen auxiliaire peut être placé sur une surface de peau, avec des étapes suivantes :
- d'application de pistes conductrices (109) réalisées par une pâte conductrice insensible par rapport à un allongement, une flexion, un étirage et/ou un pliage sur un côté supérieur d'une strate inférieure (107),
- de placement d'une strate supérieure (105) sur la strate inférieure (107) avec les pistes conductrices (109) si bien que la pâte conductrice est disposée entre la strate inférieure (107) et la strate supérieure (105),
- de disposition de plusieurs capteurs de température (111) sur un côté supérieur de la strate supérieure (105) et de raccordement de chaque capteur de température (111) entre deux pistes conductrices (109) au moyen d'un raccordement électrique (119),
- d'application d'une couche de contact (103) sur le côté supérieur de la strate supérieure (105) avec les capteurs de température (111) si bien qu'une couche de capteur (101) est réalisée, et
- d'application d'un côté inférieur de la strate inférieure (107) sur une ébauche de moyen auxiliaire (115) ou sur un moyen auxiliaire et/ou de mise en forme de l'ébauche de moyen auxiliaire avec la couche de capteur (101) raccordée si bien qu'un moyen auxiliaire est présent.

12. Procédé destiné à déterminer une différence de température relative sur une surface de peau, dans lequel une couche de capteur (101) selon l'une quelconque des revendications 1 à 9 ou un moyen auxiliaire selon la revendication 10 est utilisée ou utilisé, avec des étapes suivantes :
- de formation de la couche de capteur (101) sur la surface de peau si bien que la couche de contact (117) de la couche de chateur (101) est en contact avec la surface de peau ou avec une face textile entourant la surface de peau,
- de mesure de températures au moyen de plusieurs capteurs de température (111) dans la couche de capteur (101),
- de détermination de la différence de température relative entre les plusieurs capteurs de température (111), si bien qu'une inflammation de la surface de peau peut être identifiée de manière précoce à l'aide d'une augmentation de température locale.
